# FASCICULE DE BREVET EUROPEEN

(11) **EP 0 690 904 B1**
(45) Date de publication et mention de la délivrance du brevet: **25.06.1997**
(21) Numéro de dépôt: 94910439.2
(22) Date de dépôt: 18.03.1994
(51) Int. Cl.: C11B 3/00, C11B 7/00, A61K 7/48

(54) **MATIERES INSAPONIFIABLES D'ORIGINE VEGETALE ET COMPOSITIONS COSMETIQUES LES CONTENANT**
UNVERSEIFBARE MATERIALIEN PFLANZLICHEN URSPRUNGS UND DIESE ENTHALTENDE KOSMETISCHE PRÄPARATE
UNSAPONIFIABLE MATERIALS OF VEGETABLE ORIGIN AND COSMETIC COMPOSITIONS CONTAINING SAME

(30) Priorité: 19.03.1993 FR 9303226
(43) Date de publication de la demande: 10.01.1996
(73) Titulaire: DESLOG, F-75009 Paris (FR); Guichard, Jean-Michel, 78955 Carrières-sur-Poissy (FR)
(72) Inventeur: GUICHARD, Jean-Michel, F-78955 Carrières-sur-Poissy (FR); LAUR, Joel, F-33700 Merignac (FR); CASTERA, Anne, F-33600 Pessac (FR); MORDRET, François, F-33170 Gradignan (FR); PAGES-XATART-PARES, Xavier, F-33610 Cestas (FR)
(74) Mandataire: Portal, Gérard
(86) Numéro de dépôt international: FR9400301
(87) Numéro de publication internationale: WO9421764

(56) Documents cités:
- EP-A- 0 145 607
- FR-A- 575 541
- FR-A- 2 676 645
- GB-A- 1 511 617
- US-A- 3 012 891
- CHEMICAL ABSTRACTS, vol. 88, no. 22, 29 Mai 1978, Columbus, Ohio, US; abstract no. 154746b, 'Removal of gums from shea butter' page 121 ;colonne 2 ;
- DATABASE WPI Week 8615, Derwent Publications Ltd., London, GB; AN 86-097587 & JP,A,6 104 295 (ASAHI DENKA KOGYO) 1 Mars 1986
- DATABASE WPI Week 7105, Derwent Publications Ltd., London, GB; AN 71-09123S & JP,B,46 004 149 (KANGEAFUCHI CHEM IND CO L) 1971

## Description

La présente invention concerne un nouveau procédé de préparation de fractions de matières grasses d'origine végétale enrichies en matières insaponifiables. Elle concerne également l'utilisation de ces fractions et de leurs mélanges pour la préparation de compositions cosmétiques et/ou pharmaceutiques, notamment dermatologiques.

Les matières insaponifiables, selon la définition donnée dans la norme NF T 60-205-1 qui décrit la méthode qui permet la détermination de la teneur (méthode de référence), constituent toutes les substances présentes dans le produit qui, après saponification de celui-ci par l'hydroxyde de potassium et extraction par un solvant spécifié, ne sont pas volatiles dans les conditions opératoires spécifiées.

Ainsi, comme cela est précisé dans cette norme, les matières insaponifiables comprennent des constituants naturels des matières grasses tels les stérols, les hydrocarbures et alcools supérieurs, aliphatiques et terpéniques, ainsi que les substances organiques étrangères extraites par le solvant et non volatiles à 103°C (par exemple huiles minérales) qu'elles peuvent éventuellement contenir.

Dans le présent exposé, on désignera indifféremment par "matières insaponifiables" ou "insaponifiable" les matières insaponifiables telles qu'elles sont définies ci-dessus.

Les industries pharmaceutique et cosmétique font appel depuis les temps les plus reculés à des extraits de matières grasses d'origine végétale. Il est apparu depuis un certain nombre d'années dans ces industries que des propriétés biologiques particulièrement intéressantes résultaient de l'utilisation de matières grasses végétales ou d'extraits de matières grasses végétales riches en matières insaponifiables.

Certaines huiles végétales comme les huiles d'avocat, de soja, de colza, d'olive, et surtout de karité sont connues pour être particulièrement riches en matières insaponifiables et/ou contenir dans ces matières insaponifiables un ou plusieurs constituants intéressant les secteurs précités.

On connaît de nombreux procédés de fractionnement des matières grasses d'origine végétale visant à récupérer ou à éliminer certains de leurs composants en vue de leur utilisation dans différentes industries, en particulier dans l'industrie alimentaire et dans les industries pharmaceutique et cosmétique.

Un intérêt tout particulier a été apporté au karité.

De nombreux procédés de fractionnement de l'huile de karité ont été décrits. La plupart de ces procédés font appel à des cétones aliphatiques et proposent d'éliminer le karitène pour récupérer la fraction soluble dans la cétone et utilisable dans l'industrie du cacao comme substitut et/ou équivalent de beurre de cacao.

A la différence des procédés cités ci-dessus, mais toujours avec l'optique de récupérer un produit utilisable dans l'industrie alimentaire, le brevet US 4103 039 propose de dissoudre à chaud la graisse de karité dans l'éthanol, d'éliminer la fraction non soluble dans l'éthanol, de laisser refroidir la solution restante à une température comprise entre 20 et 60°C de façon à séparer ladite solution en deux couches distinctes liquides, la couche supérieure étant pauvre en huile et la couche inférieure riche en huile. Dans ce document, la couche riche en huile est ensuite dissoute dans un solvant organique tel que l'hexane puis soumise à une opération de fractionnement par cristallisation dans un solvant tel que l'hexane.

Ce document ne cherche nullement un enrichissement quelconque d'une des fractions en matières insaponifiables puisque son but est de produire un substitut du beurre de cacao pour usage alimentaire. Par ailleurs, le procédé décrit dans ce document doit recourir à deux solvants, à savoir l'éthanol pour insolubiliser les gommes puis l'hexane dans lequel se fait le fractionnement.

Le brevet U.S.-A- 3 012 891 vise lui aussi la préparation d'un produit de substitution du beurre de cacao destiné à un usage alimentaire et propose à cet effet de mélanger de l'huile de palme ou une fraction d'huile de palme et une fraction solide de beurre de karité, cette fraction étant obtenue après deux étapes successives de recristallisation destinées à éliminer les produits solides résultant de la première recristallisation pour ne s'intéresser qu'aux produits solides obtenus lors de la seconde recristallisation.

Selon une des ces caractéristiques essentielles, la présente invention concerne un procédé de préparation de fractions de matières grasses d'origine végétale enrichies en matières insaponifiables, caractérisé en ce qu'il consiste à traiter ladite matière grasse d'origine végétale par un solvant polaire de type cétonique de façon à récupérer d'une part une fraction insoluble à chaud (I) qui constitue une première fraction riche en matières insaponifiables et une solution dans le solvant cétonique de la fraction (S) dite fraction soluble à chaud et à soumettre ladite fraction soluble à chaud (S) à une étape de cristallisation dans un solvant, dit solvant de cristallisation, à température inférieure à 0°C, à filtrer le produit ainsi obtenu pour récupérer un filtrat (F) qui constitue, après évaporation du solvant de cristallisation, une deuxième fraction (I') riche en matières insaponifiables.

Les matières grasses susceptibles d'être traitées par le procédé selon l'invention sont toutes les matières grasses d'origine végétale.

Il peut s'agir de matières grasses brutes ou raffinées provenant de graines (par exemple, colza, soja) ou de fruits (par exemple, karité, avocat, olive) de plantes oléagineuses.

Le procédé selon l'invention peut également s'appliquer au traitement des beurres végétaux tels que le beurre d'illipé ou de Bornéo, de sal, la graisse de noyau de mangue, l'allanblackia, le suif de kokum ou goa, la graisse de dhupa, les beurres ou huiles provenant des familles des sapotacées et diptérocarpacées, ainsi que des plantes tropicales.

On choisira de préférence des matières grasses naturellement riches en matières insaponifiables et/ou des matières grasses dans lesquelles les matières insaponifiables contiennent un ou plusieurs constituants pouvant présenter des propriétés intéressantes dans le domaine de la cosmétologie, de la pharmacie, de la médecine.

Lorsque la matière grasse d'origine végétale soumise au procédé de l'invention est une matière grasse brute, elle peut être obtenue suivant des techniques classiques, en particulier des techniques de pression dans le cas de l'olive, du karité ou de l'avocat ou des techniques de pression/extraction, par exemple dans le cas du soja, de l'avocat ou du colza. Elle peut également être obtenue par des techniques artisanales, c'est le cas du beurre de karité.

La matière grasse d'origine végétale soumise au procédé de l'invention peut également être préalablement soumise à une étape de raffinage.

A titre d'exemple de procédé de raffinage, on citera les procédés classiques de raffinage chimique ou physique ou les procédés plus particuliers de raffinage du beurre de karité qui permettent de conserver en particulier le maximum de matières insaponifiables.

Le raffinage chimique avantageusement utilisé appliqué aux matières grasses végétales avant de les soumettre au procédé selon l'invention pourra être tout procédé de raffinage chimique classique, en particulier tout procédé comprenant les étapes suivantes :
- étape 1 :: étape dite de démucilagination par insolubilisation des phosphatides à l'eau généralement en présence d'acide, le plus souvent phosphorique, et séparation par décantation ou centrifugation (procédé continu)
- étape 2 :: neutralisation des acides gras libres de l'huile par addition d'une solution de soude et séparation des savons formés (appelés pâtes de neutralisa[ion) le plus souvent par centrifugation suivie de plusieurs lavages à l'eau, les étapes 1 et 2 étant souvent réalisées simultanément
- étape 3 :: décoloration par des terres décolorantes activées, à 100°C environ sous vide réduit et filtration
- étape 4 :: opération de désodorisation nécessaire pour éliminer les composés responsables des flaveurs d'une huile et produire une huile raffinée alimentaire et de bonne conservation. Ccttc opération est réalisée dans un appareil appelé désodorisateur procédant par chauffage à haute température de l'huile (180-220°C) sous un vide de l'ordre de 4 torrs (soit environ 532 Pa) et injection massive de vapeur d'eau.

On peut également soumettre au procédé selon l'invention une matière grasse végétale préalablement soumise à un procédé de raffinage physique.

Par raffinage physique, on entend en fait une variante du procédé de raffinage chimique exposé ci-dessus, la différence étant que l'étape de neutralisation à la soude n'est pas pratiquée et que l'élimination des acides gras libres de l'huile est réalisée au niveau de la désodorisation qui est pratiquée alors de façon neutralisante à plus haute température, dans un équipement adapté.

Dans le cas du karité, il est judicieux de procéder sans démucilagination par double neutralisation chimique et léger terrage dans des conditions douces de façon à minimiser les pertes en insaponifiable et/ou plusieurs de ses constituants.

Le traitement par un solvant polaire de type cétonique est de préférence réalisé à la température d'ébullition dudit solvant.

Comme solvant cétonique, on peut choisir toute cétone comprenant, au plus, 10 atomes de carbone.

A titre d'exemples de solvants cétoniques préférés selon l'invention, on citera l'acétone, la méthylisobutylcétone. On choisira de préférence l'acétone.

On déterminera en fonction de la matière grasse végétale traitée la quantité de solvant cétonique. On déterminera en particulier la quantité optimum permettant à la fois une récupération sélective des insolubles, en particulier du karitène tout en évitant d'utiliser une quantité trop importante de solvants de façon à ne pas gêner l'étape ultérieure de cristallisation.

A titre d'exemple dans le cas du traitement du beurre de karité, on utilisera pour 200 g de beurre de karité une quantité d'environ 1 l d'acétone.

Pour assurer une bonne extraction de l'ensemble des produits solubles dans le solvant cétonique, l'ébullition sera maintenue pendant un temps suffisant, avantageusement de l'ordre de 30 min, avant de séparer par décantation, puis filtration ou centrifugation la partie insoluble.

L'étape de cristallisation à température inférieure à 0°C peut être réalisée dans tout solvant de la fraction soluble à chaud dans le solvant cétonique utilisé dans la première étape du procédé selon l'invention.

A titre d'exemples de tels solvants, on citera en particulier l'acétone, la méthylisobutylcétone, l'hexane, le dichlorométhane, le dichloroéthane, l'acétate d'éthyle ou l'isopropanol.

Si le solvant utilisé pour l'étape de cristallisation est un solvant différent de celui utilisé dans l'étape précédente d'insolubilisation à chaud, on élimine préalablement, à la mise en solution dans le solvant de cristallisation, le solvant cétonique utilisé pour l'insolubilisation à chaud. Cette élimination peut être réalisée classiquement par évaporation sous vide du solvant cétonique.

Selon une variante particulièrement avantageuse du procédé selon l'invention, on utilise pour l'étape de cristallisation le même solvant que pour l'étape de dissolution à chaud.

Selon une variante particulièrement avantageuse, on réalise l'étape de cristallisation directement sur la solution cétonique récupérée après élimination de la fraction non soluble à chaud dans ce solvant.

Selon une variante particulièrement avantageuse, on utilise l'acétone comme solvant dans les deux étapes de dissolution à chaud et de cristallisation.

Selon un mode de réalisation avantageux, on utilise des dilutions pondérales de matière végétale dans le solvant cétonique comprises entre 5 et 30 %, avantageusement de l'ordre de 20 %.

A titre de solvant préféré pour cette étape, on choisira l'acétone.

La température de cristallisation sera de préférence située bien en dessous de 0°C, de préférence entre -15 et -30°C. Toutefois, des températures plus basses pourront également être utilisées.

Lorsque l'étape de cristallisation est conduite au niveau laboratoire ou pilote, en batch, la durée de cristallisation est de l'ordre de 12 à 24 h. Toutefois, l'utilisation de cristalliseur industriel permettant de fixer un barème de refroidissement jusqu'à la température voulue de cristallisation puis de maturation à cette température avant filtration permet l'optimisation du procédé et notamment la réduction de la durée de l'opération.

Après cristallisation, le produit est filtré et l'on récupère, d'une part, un filtrat (F), d'autre part, une fraction solide dite concret, contenant une partie du solvant.

Après évaporation du solvant, ce concret présente des caractéristiques physico-chimiques très proches de celle de la matière grasse végétale de départ, mise à part la teneur en matières insaponifiables qui est considérablement réduite. Ceci offre un avantage important en particulier, par exemple, dans le cas du beurre de karité, puisque cela permet de disposer d'un produit plus intéressant dans le secteur alimentaire que le beurre de karité non transfomné, notamment pour la fabrication de substituts et/ou d'équivalents de beurre de cacao où les caractéristiques rhéologiques et de fusion de la graisse, liées étroitement à la composition en acides gras et en triglycérides, sont des facteurs déterminants.

Le filtrat est ensuite soumis à une étape destinée à éliminer le solvant, par exemple une étape d'évaporation sous vide. Cette étape conduit à la récupération d'une deuxième fraction riche en insaponifiables (I').

Un avantage du procédé selon l'invention, tel qu'il a été décrit précédemment, est qu'il conduit à deux fractions riches en insaponifiables. Ces deux fractions peuvent ensuite être mélangées en totalité ou en partie pour conduire à de nouveaux mélanges enrichis en insaponifiables. Ainsi, selon une variante particulièrement avantageuse, le procédé selon l'invention comprend, en outre, une étape de mélange d'au moins une partie, de préférence de la totalité, de chacune des deux fractions enrichies en matières insaponifiables.

Ainsi, la fraction (I'), ou une partie de cette fraction, est avantageusement mélangée à la première fraction riche en insaponifiables constituée de la partie insoluble à chaud dans l'acétone (fraction I) ou à une partie de cette fraction.

Selon une variante avantageuse du procédé selon l'invention, on dissout la fraction (I) dans un solvant avant de mélanger cette dernière à la fraction (I').

A titre de solvant préféré pour dissoudre la fraction (I), on choisira un solvant de la famille des solvants chlorés du type chloroforme, chlorure de méthylène, dichlorométhane, dichloroéthane ou un solvant chlorofluoré du type flugène.

Après mélange de la fraction (I') et de la fraction (I,) ou des quantités désirées de ces fractions, et évaporation du solvant, on récupère un produit final qui constitue une fraction de la matière végétale traitée particulièrement enrichie en matières insaponifiables.

On conçoit qu'un avantage du procédé de l'invention est de permettre d'obtenir, par mélange entre les deux fractions, des fractions enrichies en insaponifiables dont on pourra modifier la teneur en insaponifiables à la fois en jouant sur les proportions des deux fractions et sur la température de recristallisation.

Ainsi, à titre d'exemple, par traitement de 3 kg d'un beurre de karité neutralisé et décoloré contenant 0,42 % d'acide oléique et 6,4 % de matières insaponifiables, on obtiendra, dans les conditions de l'exemple 6 ci-dessous, en utilisant le procédé selon l'invention et après mélange des fractions (I) et (I'), 150 g d'une fraction enrichie en matières insaponifiables contenant 2,8 % d'acide oléique et au moins 48 % de matières insaponifiables.

Les mélanges de fractions enrichies en matières insaponifiables obtenus selon le procédé de l'invention, tout particulièrement ceux obtenus à partir de karité, s'avèrent particulièrement intéressants comme principe actif de compositions cosmétiques et/ou pharmaceutiques, notamment dermatologiques.

Ainsi, selon un deuxième aspect, l'invention concerne l'utilisation des mélanges de fractions enrichies en insaponifiables décrits précédemment pour la préparation d'une composition cosmétique ou pharmaceutique, notamment dermatologique à activité antiradicalaire et/ou anti-élastase.

Suivant une variante, la composition précitée permet d'obtenir une bonne protection contre les agressions classiques auxquelles est soumise la peau, à savoir les agressions chimiques, thermiques, mécaniques et résultant de l'exposition aux radiations lumineuses visibles et ultraviolette.

Suivant une autre variante, la composition précitée est à activité antiinflammatoire.

Selon un autre de ces aspects, l'invention concerne une composition cosmétique, contenant à titre d'ingrédient actif une quantité cosmétiquement active d'un mélange de fractions enrichies en insaponifiables obtenu par traitement d'un beurre de karité selon le procédé de l'invention.

Selon un autre de ces aspects, l'invention concerne une composition pharmaceutique, notamment dermatologique, comprenant à titre d'ingrédient actif une quantité pharmaceutiquement efficace d'un mélange de fractions enrichies en insaponifiables obtenu selon le procédé de l'invention.

Dans l'un et l'autre des deux aspects précédents, on utilisera, de préférence, des fractions riches en insaponifiables obtenues par traitement du beurre de karité.

Les mélanges de fractions insaponifiables contiendront, de préférence, 18 à 50 % en poids de composés insaponifiables, de préférence 20 à 50 %, de préférence encore de l'ordre de 30 %.

Ces mélanges seront incorporés à des concentrations comprises entre 0,5 et 99 % en poids, de préférence comprises entre 2 et 60 % dans un excipient véhicule ou support cosmétiquement ou pharmaceutiquement acceptable.

Comme on l'a vu précédemment, l'intérêt des compositions cosmétiques ou pharmaceutiques, notamment dermatologiques, utilisant les mélanges de fractions enrichies en insaponifiablc obtenus suivant le procédé précédemment décrit résulte principalement de deux avantages :

1. la ou les fractions enrichies obtenues se présentent sous un aspect identique à celui d'une graisse naturelle semi-concrète, facile à mettre en oeuvre et de bel aspect,

2. elles présentent, du fait de leur grande richesse en insaponifiablc, une bonne protection contre les agressions classiques auxquelles est soumise la peau, à savoir les agressions chimiques, thermiques, mécaniques et résultant de l'exposition aux radiations lumineuses visibles et ultraviolettes.

Ainsi, de bonnes propriétés antiradicalaires, largement supérieures à celles des graisses de départ non enrichies en insaponifiable par le procédé décrit précédemment, ont notamment été mises en évidence sur des émulsions réalisées avec les mélanges précités et les mélanges eux-mêmes par des tests réalisés in vitro.

Pour mettre en évidence l'activité antiradicalaire, on a utilisé avantageusement le test dit test "hématies" dont le principe est décrit ci-dessous et qui permet d'évaluer, in vitro, le pouvoir antiradicalaire d'une molécule. On suit son pouvoir d'inhibition de l'hémolyse d'érythrocytes soumis à une attaque radicalaire.

On utilise, pour ce test, un générateur hydrosoluble de radicaux libres : le 2,2' azobis-diamidinopropane hydrochlorure.

Plus précisément, ce test consiste à soumettre des hématies isolées de leur plasma à une agression de type oxydatif, dans des conditions contrôlées et standardisées permettant la mise en jeu de tout leur équipement enzymatique et moléculaire pour résister à cette agression jusqu'à ce que la membrane cellulaire soit modifiée, puis l'éclatement et la lyse de la cellule.

La mesure de l'hémoglobine est alors réalisée par spectrophotométrie.

La résistance de la population d'hématies testées s'exprime alors par le temps de libération de 50 % du contenu en hémoglobine (T50)

On calcule un pourcentage de protection de l'hémolyse. Le calcul se fait en comparant T50 (50 % d'hémolyse) des essais, à celui du témoin.

Plus le T50 d'un essai est élevé, plus la protection est importante ; l'hémolyse a été retardée. Au contraire, un T50 faible signifie que la molécule est plutôt pro-oxydante.

Ainsi avec les mélanges enrichis en insaponifiable obtenus selon l'invention, on atteint des valeurs de T50 de 128 à 196.

De la même façon, de bonnes propriétés anti-élastases également supérieures à celles des graisses de départ non enrichies en composés insaponifiables, par le procédé décrit précédemment, ont été mises en évidence sur les mélanges de l'invention, en particulier au moyen des tests in vitro décrits ci-dessous et permettant de mettre en évidence l'inhibition de l'élastase leucocytaire humaine (ELH).

Les deux tests suivants ont principalement été utilisés.

### a) Utilisation du substrat synthétique spécifique de l'ELH ; le N-méthoxysuccinyl-(ala)₂-pro-val-paranitroanilide

Selon ce test, l'hydrolyse du substrat par l'élastase leucocytaire est suivie par spectrophotométrie pendant cinq minutes.

Différentes concentrations d'inhibiteurs sont incubées dans le tampon de réaction en présence d'enzyme, puis le substrat est ajouté.

Les pourcentages d'inhibition sont calculés par rapport à une courbe témoin : hydrolyse du substrat en l'absence d'inhibiteur.

On mesure également la concentration en inhibiteur pour laquelle on obtient 50 % d'inhibition de l'enzyme (=IC 50).

### b) Utilisation du substrat naturel de l'ELH ; élastine fibreuse :

Ce test correspond à des conditions plus physiologiques.

Selon ce test, différentes concentrations d'inhibiteurs sont incubées dans le tampon de réaction, en présence de l'enzyme, puis 150 µg d'élastine fibreuse radiomarquée sont ajoutés.

Les échantillons sont incubés à 37°C sous agitation. L'hydrolyse du substrat est mesurée après 1, 2, 4 et 6 heures d'incubation.

On sépare l'élastine non digérée (insoluble) de l'élastine hydrolysée (soluble), par centrifugation à 10 000 rpm pendant 30 secondes. La radio-activité provenant de l'hydrolyse de l'élastine est mesurée dans 50 µl de surnageant.

De même que pour l'analyse avec le substrat synthétique, on mesure les pourcentages d'inhibition et les IC 50.

Les mélanges enrichis en insaponifiable selon l'invention conduisent à des pourcentages d'inhibition de l'élastase leucocytaire humaine de 16 à 80 %.

Les techniques évoquées ci-dessus permettent de savoir si la ou les molécules possèdent un intérêt dans le cadre de l'inflammation. En effet, lors de pathologie inflammatoire, il y a production locale de divers radicaux libres et augmentation de la synthèse de l'élastase leucocytaire humaine. Ces tests sont réalisés in vitro mais sont adaptables in-vivo ou ex-vivo ; ils sont reconnus comme méthodes d'objectivation cosmétique.

Selon une variante avantageuse de l'invention, les compositions cosmétiques et/ou pharmaceutiques, notamment dermatologiques, peuvent en outre contenir d'autres composants connus pour leur action de protection contre les agressions de la peau et qui agissent de façon snergique avec les mélanges riches en insaponifiables utilisés selon l'invention.

Ainsi, les composants suivants sont avantageusement associés à :
- des tocophérols ou des tocotriénols, notamment la vitamine E,
- des acides gras polyinsaturés de la série n-3 et n-6 ou des huiles ou extraits végétaux en contenant,
- des glycolipides tels que céramides, cérébrosides, glycosylcéramides, d'origine végétale ou animale.

Ces composants sont dissous généralement dans la phase lipophile avant préparation de la composition cosmétique.

Plus précisément :
- Les tocophérols et les tocotriènols sont des 8-méthylchroman-6-ols substitués en 1 par un groupe méthyle et une chaîne de type polyisoprénique à seize atomes de carbone saturée (tocophérols) ou triinsaturée (tocotriénols) ; les variétés alpha, bêta, gamma, delta différent entre elles par le nombre et la localisation des groupes méthyle en 5 et 7.

Les tocophérols et tocotriènols représentent généralement 200 à 1 200 mg par kg d'huile végétale non raffinée et sont également présents dans certaines huiles animales (poisson).

Les tocophérols sont classiquement présents dans de nombreuses huiles et graisses, tournesol en particulier.

Les tocotriènols sont plus rarement rencontrés, mais sont notamment présents dans les huiles, en particulier les huiles de germe de blé et de palme.

Ces constituants présentent des propriétés antioxydantes reconnues. L'alpha tocophérol est plus connu sous le nom de vitamine E.
- Les acides gras polyinsaturés de la famille n-3 et n-6 sont, conformément à la nomenclature, des acides acide gras présentant plusieurs insaturations sur leur chaîne carbonée.

En outre, pour la famille n-3 (respectivement n-6) la première double liaison est située entre le 3ème (respectivement 6ème) et le 4ème atome de carbone (respectivement 7ème) compté à partir du groupe méthyle de la chaîne carbonée.

Les acides gras les plus courants parmi la famille n-6 sont l'acide linoléique C18:2 (n-6, 9), gamma-linolénique C18:3 (n-6, 9, 12) et l'acide arachidonique C20:4 (n-6, 9, 12, 15).

Les acides gras les plus courants de la famille n-3 sont l'acide alpha-linolénique C18:3 (n-3, 6, 9), l'acide eïcosapentaénoïque ou EPA, C20:5 (n-3, 6, 9, 12, 15) et l'acide docosahexaénique ou DHA, C22:6(n-3, 6, 9, 12, 15, 18).
- Les glycolipides désignent une fraction intéressante des lipides polaires extraits de certaines plantes supérieures (feuilles, racines, graines, algues, levures, champignon) ; le blé est notamment une source reconnue et contient environ 3 % de glycolipides par rapport aux lipides totaux.

Parmi les nombreux constituants de cette famille, on choisira :
. les céramides : combinaison d'un aminoalcool de type sphingosine avec un acide gras lié à la fonction amine formant ainsi une amide,
. les céréhrosides ou glycosylcéramides : céramides dont la fonction alcool primaire est éthérifiée avec un sucre (céramides monohexosides, polyhexosides).

Ces sphingolipides (céramides et cérébrosides) sont dotés de diverses propriétés reconnues dont une activité antiradicalaire, un pouvoir anti-élastasique et une action hydratante.

Les exemples suivants sont donnés à titre non limitatif et purement illustratif de l'invention.

### Exemples

Sauf indication contraire, les pourcentages indiqués dans les résultats et compositions ci-dessous sont exprimés en poids.

Dans tous les exemples, le dosage des matières insaponifiables est réalisé selon la norme AFNOR NFT 60205-1, méthode de référence par extraction à l'oxyde diéthylique après saponification en milieu éthanolique.

L'acidité oléique est déterminée selon la norme AFNOR NF T 60-204 selon la méthode au mélange oxyde diéthylique-éthanol.

Les séparations par chromatographie sur couche mince sont réalisées sur plaques de gel de silice (Type 60, MERCK) de 0,25 mm d'épaisseur. Le solvant d'élution est un mélange hexane/acétate d'éthyle 80:20 (v/v) et les taches sont révélées par pulvérisation d'une solution d'H₂SO₄ à 50 % et minéralisation à 150°C.

Les constituants sont identifiés par comparaison des distances de rétention avec celles de produits de référence : alcool oléique, cholestérol, squalène, eicosène, β-amyrine, triglycérides, acide oléique.

La détermination de la composition en acides gras libres, monoglycérides, diglycérides, triglycérides et stérols, est réalisée par chromatographie en phase gazeuse sur colonne capillaire courte (7m) avec phase stationnaire apolaire SE30 et après dérivation des composés sous forme T.M.S.

L'injection est du type "sur colonne", le détecteur FID est réglé à 350°C et l'analyse est réalisée en programmation de température (100 à 340°C).

Les constituants sont identifiés à partir de leur temps de rétention.

Les résultats sont normalisés à 100 (composition centésimale).

### Exemple 1

### a) Essai 1 :

On dissout 50 g de beurre de karité contenant 7 % d'insaponifiable dans 500 ml d'acétone. On porte l'acétone à ébullition pendant 15 min et on récupère un résidu (I) non soluble dans l'acétone à chaud ainsi qu'une solution (S) dans l'acétone et on la refroidit à -20°C.

Le mélange (S) est maintenu à -20°C pendant 12 h.

Après précipitation, le surnageant est filtré.

Le précipité est rincé par plusieurs volumes d'acétone (à froid). Le filtrat est alors séché après évaporation sous vide du solvant, puis pesé.

Le tableau I donne les résultats de cet essai.

L'analyse du résidu (I) montre que cette fraction insoluble à chaud représente des constituants insaponifiables fortement apolaires du type gommes, latex, contenant entre autres le karitène. Cette fraction insoluble à chaud est mélangée avec la fraction soluble à froid.

### b) Essai 2 :

On opère comme dans l'essai 1, mais à partir de 100 g de beurre de karité dissous dans 500 ml d'acétone.

Les résultats sont consignés dans le tableau i.

### c) Essai 3:

On opère comme dans l'essai 1, mais la cristallisation est effectuée à -10°C,

Les résultats sont consignés dans le tableau I.

**TABLEAU I**

| n° essai | Rendement en fraction soluble dans l'acétone | Teneur en insaponifiable de la fraction soluble dans l'acétone (pureté) | Poids d'insaponifiable obtenu (P1) | Poids total théorique d'insaponifiable (P2) | Rendement en insaponifiable (P1/P2x100) % |
|---|---|---|---|---|---|
| 1 | 18,2 % | 22,95 % | 2,1 g | 3,5 g | 60,0 % |
| 2 | 14,7 % | 23,60 % | 3,5 g | 7 g | 49,6 % |
| 3 | 24,5 % | 16,30 % | 2,0 g | 3,5 g | 57,1 % |

### Exemple 2

On procède comme dans l'exemple 1 et on réalise les 3 essais ci-dessous notés 4, 5, 6 montrant l'effet de la dilution du beurre de karité dans l'acétone. Dans ces essais, la phase de fractionnement par le froid de la fraction soluble à chaud est réalisée à -20°C.

Les résultats sont consignés dans le tableau II ci-dessous où les pourcentages sont donnés en poids.

**TABLEAU II**

| **Choix de la dilution** | | | | |
|---|---|---|---|---|
| (Acétone, température : -20°C) | | | | |
| n° essai | Dilution (%) | Rendement en fraction soluble (%) | Teneur en insaponifiable de la fraction soluble (%) | Rendement en insaponifiable (*) |
| 4 | 5 | 16,0 | 20,6 | 47,1 |
| 5 | 10 | 18,2 | 23,0 | 59,8 |
| 6 | 20 | 14,7 | 23,6 | 49,6 |

| | | | | |
|---|---|---|---|---|
| (*) : (poids obtenu/poids théorique x 100) % | | | | |

### Exemple 3

On procède comme dans les exemples précédents, et on réalise les essais ci-dessous (7, 8, 9) destinés à montrer l'influence de la température de fractionnement par le froid sur le rendement en fraction insaponifiable soluble à froid (F), la teneur en insaponifiable de cette fraction soluble et le rendement en insaponifiable de cette étape.

**TABLEAU III**

| n° essai | Température (°C) | Rendement en fraction soluble (%) | Teneur en insaponifiable de la fraction soluble (%) | Rendement en insaponifiable (*) |
|---|---|---|---|---|
| 7 | -10°C | 24,5 | 16,3 | 57,1 |
| 8 | -20°C | 13,1 | 22,5 | 42,1 |
| 9 | -30°C | 7,7 | 37,2 | 40,9 |

| | | | | |
|---|---|---|---|---|
| (*) : (poids obtenu/poids théorique x 100) % | | | | |

La température de cristallisation est un facteur très influent sur l'efficacité du fractionnement.

L'abaissement de la température à -30°C permet d'obtenir une fraction à plus de 35 % de pureté en matières insaponifiables. Soit, ici, un enrichissement d'un facteur 5 par rapport au beurre brut de départ.

### Exemple 4

Dans les essais 10, 11, 12 suivants, on traite, selon le procédé de l'invention, trois qualités différentes de beurre de karité dont les caractéristiques sont données dans le tableau IV ci-dessous :

**TABLEAU IV**

| | Acidité oléique (%) | Teneur en insaponifiable (%) |
|---|---|---|
| Beurre brut | 5,3 | 7,0 |
| Beurre neutralisé | 0,37 | 6,1 |
| Beurre raffiné | 0,13 | 6,6 |

Les résultats obtenus sont données dans le tableau V.

**TABLEAU V**

| Essais de cristallisation dans l'acétone sur différentes matières d'oeuvre | | | | |
|---|---|---|---|---|
| n° essai | Matière d'oeuvre (Acidité oléique) | Rendement en fraction soluble (%) | Acidité oléique de la fraction soluble (%) | Teneur en insaponifiable de la fraction soluble (%) |
| 10 | beurre brut (*) | 13,1 | 20,3 | 22,5 |
| 11 | beurre neutralisé (0,37) (**) | 13,5 | ≈ 2,0 | 23,3 |
| 12 | beurre raffiné (0,13) (*) | 11,5 | 0,9 | 25,9 |

| | | | | |
|---|---|---|---|---|
| (*) : dilution : 10 %, température ; -20°C | | | | |
| (**) : dilution : 20 %, température : -15°C. | | | | |

Ces essais montrent que la pureté en insaponifiable de la fraction soluble dans l'acétone est peu dépendante de la nature du produit de départ (brut, semi-raffiné ou raffiné).

### Exemple 5

Deux beurres de karité bruts (A et B) et un beurre neutralisé et décoloré (C) ont été choisis. Leurs caractéristiques physico-chimiques sont données dans le tableau VI. L'insaponifiablc a été analysé par chromatographie sur couche mince afin de mettre en évidence la proportion plus ou moins importante de constituants apolaires : "gommes", karitènes. Les résultats senti-quantitatifs sont donnés dans le tableau VI où "+" signifie "présence" et "+++" signifie "très forte présence".

**TABLEAU VI**

| Nature de l'échantillon | Acidité oléique (%) | Teneur en insaponifiable (%) | Analyse CCM de l'insaponifiable | |
|---|---|---|---|---|
| | | | Stérols alcools | "Gommes karitènes" |
| A | 5,3 | 6,4 | + | +++ |
| B | 6,5 | 8,4 | + | +++ |
| C | 0,42 | 6,4 | + | ++ |

On a réalisé unc cristallisation à -30°C, sur une prise d'essai de 50 g, avcc une dilution de 10 % dans l'acétone.

La fraction insoluble à chaud dans l'acétone (gommes) a été récupérée séparément par solubilisation à chaud dans le chloroforme, puis additionnée à la fraction soluble à froid.

Les résultats obtenus sont donnés dans le tableau VII correspondant aux essais 13, 14, 15 réalisés respectivement avec les beurres A, B, C ci-dessus.

**TABLEAU VII**

| Essais de cristallisation à -30°C dans l'acétone (dilution : 10 %) | | | | | | |
|---|---|---|---|---|---|---|
| n° essai | Matière d'oeuvre | Nature de la fraction | Teneur par rapport au beurre (%) | | Acidité oléique (%) | Teneur en insaponifiable (%) |
| 13 | A | insoluble à chaud (gommes) | 5,8 | 17,2 | 18,7 | ≈ 35 |
| | | Soluble à froid (insaponifiable) | 11,4 | | | |
| | | Insoluble à froid (huile) | 82,8 | | 2,8 | 1,4 |
| 14 | B | Insoluble à chaud (gommes) | 7,4 | 20,6 | 17,1 | ≈ 46 |
| | | Soluble à froid (insapo.) | 13,2 | | | |
| | | Insoluble à froid (huile) | 79,4 | | 3,6 | 1,7 |
| 15 | C | Insoluble à chaud (gommes) | 5,5 | 13,4 | 2,4 | ≈ 34 |
| | | Soluble à froid (insapo.) | 7,9 | | | |
| | | Insoluble à froid (huile) | 86,6 | | 0,2 | 1,9 |

Les résultats obtenus sont tout à fait cohérents.

Les teneurs en matières insaponifiables atteintes sont supérieures à 30 % et les fractions enrichies en matières insaponifiables correspondent à + de 10 % de beurre de karité de départ.

On note que l'acidité libre de la fraction obtenue à partir du beurre neutralisé décoloré est d'environ 2 %.

Les puretés en insaponifiable montrent que l'on atteint un rendement >70 % pour tous les essais, ce qui montre l'efficacité du recyclage de la fraction insoluble à chaud (gommes).

Les détemiinations de la teneur exacte en insaponifiable selon la norme sont délicates du fait justement de la présence de ces gommes non solubles dans la phase solvant (insaponifiable) et dans la phase hydro-alcoolique (savons d'acides gras).

De ce fait, les valeurs trouvées peuvent être sous-évaluées, même sur le beurre de karité tel quel.

L'analyse par chromatographie sur couche mince montre que
- les fractions insolubles à froid sont quasi-exclusivement constituées de triglycérides, il apparaît des traces d'insaponifiable (alcools gras et stérols) ;
- les fractions résultant du mélange des insolubles à chaud et solubles à froid sont constituées par ordre de polarité décroissante : de stérols, d'acides gras libres, d'alcools gras aliphatiques et triterpéniques, de triglycérides et de constituants très apolaires. Ces derniers correspondent à la fraction insoluble à chaud, c'est-à-dire aux karitènes ou gommes.

La même analyse CCM réalisée sur les matières insaponifiables montre que les matières insaponifiables restant à environ 1,5-2 % dans la fraction insoluble à froid sont constituées par des alcools et des stérols. Il ne reste plus de gommes, alors que la partie insaponifiable concentrée dans la fraction soluble à froid contient les alcools gras, les stérols et les gommes.

L'analyse par chromatographie en phase gazeuse permet d'obtenir la composition centésimale des différents constituants des fractions, en absence des gommes, dont le poids moléculaire très élevé n'est pas compatible avec une chromatographie en phase gazeuse.

Les constituants chromatographiables sont :
- les triglycérides,
- les acides gras libres,
- les glycérides partiels,
- l'insaponifiable : stérols et alcools gras.

Cette analyse confirme :
. la quasi-absence de matières insaponifiables dans les fractions insolubles à froid.
. la concentration dans les fractions enrichies en matières insaponifiables des acides gras libres, des glycérides partiels, avec présence de triglycérides.

Les compositions centésimales obtenues sont données dans le tableau VIII.

**TABLEAU VIII**

| Compositions centérimales (%) des différentes fractions obtenues par CPG sur phase stationnaire apolaire SE₃₀ (gommes ou karitènes non chromatographiées) | | | | | | |
|---|---|---|---|---|---|---|
| Réf. éch. | Réf. fraction | Acides gras libres | Monoglycérides | Diglycérides | Triglycérides | Insaponifiable "stérols+ alcools" |
| A | Insoluble à chaud + soluble à froid | 24,5 | 0,2 | 22,1 | 41,0 | 9,8 |
| | Insoluble à froid | 7,4 | 0,2 | 8,3 | 82,0 | 2,1 |
| B | Insoluble à chaud + soluble à froid | 23,2 | 0,7 | 23,5 | 42,5 | 10,2 |
| | Insoluble à froid | 4,0 | <0,1 | 2,2 | 93,6 | 0,2 |
| C | Insoluble à chaud + soluble à froid | 2,0 | 0,1 | 28,4 | 57,2 | 12,3 |
| | Insoluble à froid | 0,3 | <0,1 | 1,7 | 97,7 | 0,3 |
| Témoin: * | | 3,1 | <0,1 | 1,6 | 95,2 | 0,1 |

| | | | | | | |
|---|---|---|---|---|---|---|
| * Ce produit est le beurre de karité brut, tel quel, non fractionné, tel que décrit dans le tableau IV. | | | | | | |

Par ailleurs, les analyses par CLHP confirment que
1) la fraction insoluble à chaud correspond aux karitènes (gommes).
2) les fractions enrichies en insaponifiable contiennent tous les constituants natifs de l'insaponifiable de karité :
   * karitènes (gommes),
   * alcools triterpéniques,
   * stérols.

### Exemple 6

Un essai de cristallisation a été réalisé sur 3 kg de beurre de karité neutralisé, décoloré répondant aux spécifications de produit C de l'exemple 5.

Les conditions opératoires suivies sont les suivantes :
- prise d'essai : 3 x 1 kg,
- réalisation en 3 batchs de 5 x 200 g,
- volume acétone : 1 I pour 200 g, soit une dilution de 20 %,
- solubilisation du beurre de karité à chaud (ébullition) sous agitation.
- décantation 15 min,
- récupération de la fraction insoluble (gommes) au chloroforme,
- cristallisation : -30°C, 12 h,
- filtration (sans rinçage du précipité),
- évaporation de l'acétone,
- mélange des fractions (insolubles à chaud) + (soluble à froid) dans le chloroforme,
- évaporation + séchage,

Les résultats obtenus sont les suivants :
- poids de fraction enrichie en matières insaponifiables : environ 150 g,
- rendement en fraction enrichie en matières insaponifiables : 5 %,
- aspect de la fraction enrichie en matières insaponifiables ; huile épaisse jaune,
- pureté en matières insaponifiables de la fraction enrichie en matières insaponifiables, supérieure à 48 %.

### Exemple 7

### Composition anhydre (sous forme de baume)

On prépare par simple mélange des constituants indiqués ci-dessous à une température comprise entre 40 et 50° C des compositions sous forme de baume de composition pondérale donnée ci-dessous :
Fraction de beurre de karité enrichie en insaponifiable : 1-60 %, de préférence 5 %
vaseline : 30-60 %
cire de paraffine : 0-30 %
parfum : 1-3 %
vitamine E: 0-1 %
glycolipides ou sphingolipides : 0-2 %
huile de poisson : 0-1 %
dans lesquelles la fraction de beurre de karité enrichie en insaponifiable est constituée du mélange des fractions respectivement insolubles à chaud (I) et soluble à froid (I') obtenu dans chacun des exemples 1 à 6.

### Exemple 8

### Huile pour le visage et pour le corps

Les compositions suivantes sont préparées à partir des mêmes fractions enrichies en insaponifiable que dans l'exemple 7 :
fraction de beurre de karité enrichie : 2-10 , de préférence 5 %
huile de vaseline : 90-95
huile de poisson : 1 %
vitamine E : 1 %
glycolipides ou sphingolipides : 0-2 %

### Exemple 9

### Crème de soin émulsion eau-dans-huile

Les compositions suivantes sont préparées à partir des mêmes fractions enrichies en insaponifiable que dans l'exemple 7 :
fraction de beurre de karité enrichi 10-25 , de préférence 15 %
huile de vaseline : 10-15 %
alcool cétylique : 4 %
stéarate de sorbitan : 3 %
vitamine E: 0-1 %
huile de poisson : 0-1 %
glycolipides ou sphingolipides : 0-2 %
eau, parfum, conservateurs qsp 100

### Exemple 10

### Crème de soin émulsion huile-dans-eau

Les compositions suivantes sont préparées à partir des mêmes fractions enrichies en insaponifiable que dans l'exemple 7 :
fraction de beurre de karité : 10-20 % , de préférence 15 %
alcool cétylique : 3 %
triéthanolamine : 1 %
stéarate de glycérol : 3 %
colorant : 3 %
vitamine E : 0-1 *%*
glycolipides ou sphingolipidcs : 0-2 %
huile de poisson : 0-1 %
eau, parfum, conservateurs qsp 100

## Revendications

1. Procédé de préparation de fractions de matières grasses d'origine végétale enrichies en matières insaponifiables, caractérisé en ce qu'il consiste à traiter ladite matière grasse d'origine végétale par un solvant polaire de type cétonique de façon à récupérer, d'une part, une fraction insoluble à chaud (I) dans ledit solvant cétonique qui constitue une première fraction riche en matières insaponifiables et une solution dans le solvant cétonique d'une fraction (S) dite fraction soluble à chaud et à soumettre ladite fraction soluble à chaud (S) à une étape de cristallisation dans un solvant dit solvant de cristallisation à température inférieure à 0°C, à filtrer le produit ainsi obtenu pour récupérer un filtrat (F) qui constitue, après évaporation du solvant de cristallisation, une deuxième fraction (I') riche en matières insaponifiables.

2. Procédé selon la revendication 1, caractérisé en ce que le traitement par le solvant cétonique est réalisé à la température d'ébullition dudit solvant.

3. Procédé selon l'une des revendications 1 à 2, caractérisé en ce que le solvant cétonique est une cétone renfermant au plus dix atomes de carbone.

4. Procédé selon l'une des revendications 1 à 3, caractérisé en ce que le solvant cétonique est l'acétone.

5. Procédé selon l'une des revendications 1 à 4, caractérisé en ce que l'étape de cristallisation est réalisée dans tout solvant de la fraction soluble à chaud dans le solvant cétonique.

6. Procédé selon la revendication 5, caractérisé en ce que l'étape de cristallisation est réalisée directement sur la solution dans le solvant cétonique récupérée à l'issue de l'étape de traitement à chaud de ladite matière grasse végétale par ledit solvant cétonique.

7. Procédé selon la revendication 6, caractérisé en ce que le solvant cétonique est l'acétone.

8. Procédé selon l'une des revendications 1 à 7, caractérisé en ce que la cristallisation est réalisée à une température inférieure à -15°C, de préférence comprise entre -15 et -30°C.

9. Procédé selon l'une des revendications 1 à 8, caractérisé en ce que la matière grasse de départ est une matière grasse provenant de graines ou de fruits de plantes oléagineuses, obtenue par des techniques classiques de pression ou de pression/extraction, ladite matière grasse pouvant être soit brute, soit préalablement raffinée.

10. Procédé selon l'une des revendications 1 à 9, caractérisé en ce que la matière grasse est le beurre de karité.

11. Procédé selon la revendication 10, caractérisé en ce que le beurre de karité est brut.

12. Procédé selon la revendication 10, caractérisé en ce que le beurre de karité a subi une opération de raffinage destinée à conserver le maximum d'insaponifiable et à préserver ses constituants

13. Procédé selon l'une des revendications 1 à 12, caractérisé en ce qu'il comprend en outre une étape de mélange d'au moins une partie, de préférence de la totalité, de chacune des deux fractions (I et I') riches en matières insaponifiables.

14. Utilisation des mélanges de fractions enrichies en insaponifiables obtenus en traitant un beurre karité selon le procédé de la revendication 13 pour la préparation d'une composition cosmétique ou pharmaceutique, notamment dermatologique, à activité antiradicalaire et/ou anti-élastase.

15. Utilisation selon la revendication 14, caractérisée en ce que ladite composition permet d'obtenir une bonne protection contre les agressions classiques auxquelles est soumise la peau, à savoir les agressions chimiques, thermiques, mécaniques et résultant de l'exposition aux radiations lumineuses visibles et ultraviolettes.

16. Composition cosmétique contenant, à titre d'ingrédient actif, une quantité cosmétiquement efficace d'un mélange de fractions enrichies en composés insaponifiables obtenu par traitement d'un beurre de karité selon le procédé décrit dans la revendication 13.

17. Composition pharmaceutique, notamment dermatologique, contenant, à titre d'ingrédient actif, une quantité pharmaceutiquement efficace d'un mélange de fractions enrichies en composés insaponifiables obtenu par traitement d'un beurre de karité selon le procédé décrit dans la revendication 13.

18. Composition selon l'une des revendications 16 ou 17, caractérisée en ce que ledit mélange de fractions enrichies en composés insaponifiables contient 18 à 50 % en poids de composés insaponifiables, de préférence 20 à 50 %.

19. Composition selon l'une des revendications 16 à 18, caractérisée en ce que lesdits mélanges sont incorporés à des concentrations comprises entre 0,5 et 99 % en poids, de préférence entre 2 et 60 % dans un excipient, véhicule ou support cosmétiquement ou pharmaceutiquement acceptable.

20. Composition selon l'une des revendications 16 à 19, caractérisée en ce qu'elle contient, en outre, au moins un produit choisi dans le groupe constitué :
- des tocophérols ou des tocotriénols, notamment la vitamine E,
- des acides gras polyinsaturés de la série n-3 et n-6, des huiles ou extraits végétaux en contenant,
- des glycolipides et des sphingolipides tels que céramides, cérébrosides, glycosylcéramides, d'origine végétale ou animale.

## Patentansprüche

1. Verfahren zur Herstellung von Fraktionen von Fetten pflanzlichen Ursprungs, die an nicht-verseifbaren Materialien angereichert sind, dadurch gekennzeichnet, daß es darin besteht, daß man das genannte Fett pflanzlichen Ursprungs mit einem polaren Lösungsmittel vom Keton-Typ behandelt, um einerseits eine in dem genannten Keton-Lösungsmittel in der Wärme unlösliche Fraktion (I), die eine erste, an nicht-verseifbaren Materialien reiche Fraktion darstellt, und andererseits eine Lösung einer in der Wärme löslichen Fraktion (S) in dem Keton-Lösungsmittel zu gewinnen, und die genannte in der Wärme lösliche Fraktion (S) in einem als Kristallisations-Lösungsmittel bezeichneten Lösungsmittel bei einer Temperatur unter 0°C einer Kristallisation unterwirft, und das dabei erhaltene Produkt filtriert unter Gewinnung eines Filtrats (F), das nach dem Verdampfen des Kristallisations-Lösungsmittels eine an nicht-verseifbaren Materialien reiche zweite Fraktion (I') darstellt.

2. Verfahren nach Anspruch 1, dadurch gekennzeichnet, daß die Behandlung mit dem Keton-Lösungsmittel bei der Siedetemperatur des genannten Lösungsmittels durchgeführt wird.

3. Verfahren nach einem der Ansprüche 1 bis 2, dadurch gekennzeichnet, daß es sich bei dem Keton-Lösungsmittel um ein Keton handelt, das höchstens 10 Kohlenstoffatome enthält.

4. Verfahren nach einem der Ansprüche 1 bis 3, dadurch gekennzeichnet, daß es sich bei dem Keton-Lösungsmittel um Aceton handelt.

5. Verfahren nach einem der Ansprüche 1 bis 4, dadurch gekennzeichnet, daß die Kristallisation in jedem Lösungsmittel für die in der Wärme in dem Keton-Lösungsmittel lösliche Fraktion durchgeführt wird.

6. Verfahren nach Anspruch 5, dadurch gekennzeichnet, daß die Kristallisation direkt in der Lösung in dem Keton-Lösungsmittel, die nach der Wärmebehandlung des genannten Fettes pflanzlichen Ursprungs mit dem genannten Keton-Lösungsmittel erhalten worden ist, durchgeführt wird.

7. Verfahren nach Anspruch 6, dadurch gekennzeichnet, daß es sich bei dem Keton-Lösungsmittel um Aceton handelt.

8. Verfahren nach einem der Ansprüche 1 bis 7, dadurch gekennzeichnet, daß die Kristallisation bei einer Temperatur unter -15°C, vorzugsweise zwischen -15 und -30°C, durchgeführt wird.

9. Verfahren nach einem der Ansprüche 1 bis 8, dadurch gekennzeichnet, daß das Ausgangs-Fett ein Fett ist, das aus Samenkörnern oder Früchten von ölhaltigen Pflanzen stammt, das durch klassische Pressungs- oder Pressungs/Extraktions-Verfahren erhalten wurde, wobei das genannte Fett roh oder vorher raffiniert sein kann.

10. Verfahren nach einem der Ansprüche 1 bis 9, dadurch gekennzeichnet, daß es sich bei dem Fett um Karité-Butter handelt.

11. Verfahren nach Anspruch 10, dadurch gekennzeichnet, daß die Karité-Butter roh ist.

12. Verfahren nach Anspruch 10, dadurch gekennzeichnet, daß die Karité-Butter einer Raffinierung unterworfen worden ist, um den maximalen Gehalt an nicht-verseifbarem Material zu konservieren und ihre Bestandteile zu schützen.

13. Verfahren nach einem der Ansprüche 1 bis 12, dadurch gekennzeichnet, daß es außerdem eine Stufe umfaßt, in der mindestens ein Teil, vorzugsweise die Gesamtmenge, jeder der beiden Fraktionen (I und I'), die reich an nicht-verseifbaren Materialien sind, miteinander gemischt werden.

14. Verwendung der Mischungen von an nicht-verseifbaren Materialien angereicherten Fraktionen, wie sie bei der Behandlung einer Karité-Butter nach dem Verfahren nach Anspruch 13 erhalten werden, zur Herstellung einer kosmetischen oder pharmazeutischen, insbesondere einer dermatologischen Zusammensetzung mit einer Antiradikal- und/oder Antielastase-Aktivität.

15. Verwendung nach Anspruch 14, dadurch gekennzeichnet, daß die genannte Zusammensetzung die Erzielung eines guten Schutzes gegen die klassischen Angriffe erlaubt, denen die Haut ausgesetzt ist, d.h. gegen chemische, thermische, mechanische Angriffe und solchen Angriffen, die aus dem Einwirkenlassen von sichtbaren und ultravioletten Strahlen resultieren.

16. Kosmetische Zusammensetzung, die als aktiven Bestandteil (Wirkstoff) eine kosmetisch wirksame Menge einer Mischung von Fraktionen enthält, die an nicht verseifbaren Verbindungen angereichert sind und erhalten wurde durch Behandlung einer Karité-Butter nach dem Verfahren nach Anspruch 13.

17. Pharmazeutische Zusammensetzung, insbesondere dermatologische Zusammensetzung, die als aktiven Bestandteil (Wirkstoff) eine pharmazeutisch wirksame Menge einer Mischung von Fraktionen enthält, die an nicht-verseifbaren Verbindungen angereichert sind, die erhalten wurde durch Behandlung einer Karité-Butter nach dem Verfahren nach Anspruch 13.

18. Zusammensetzung nach einem der Ansprüche 16 oder 17, dadurch gekennzeichnet, daß die genannte Mischung von Fraktionen, die an nicht-verseifbaren Verbindungen angereichert sind, 18 bis 50 Gew.-%, vorzugsweise 20 bis 50 Gew.-% nicht-verseifbare Verbindungen enthält.

19. Zusammensetzung nach einem der Ansprüche 16 bis 18, dadurch gekennzeichnet, daß die genannten Mischungen in Konzentrationen zwischen 0,5 und 99 Gew.-%, vorzugsweise zwischen 2 und 60 Gew.-%, einem kosmetisch oder pharmazeutisch akzeptablen Exzipienten, Vehiculum oder Träger einverleibt worden sind.

20. Zusammensetzung nach einem der Ansprüche 16 bis 19, dadurch gekennzeichnet, daß sie außerdem mindestens ein Produkt enthält, das ausgewählt wird aus der Gruppe
- der Tocopherole oder Tocotrienole, insbesondere Vitamin E,
- der mehrfach ungesättigten Fettsäuren der Reihe n-3 und n-6, der Öle oder Pflanzenextrakte, die sie enthalten, und
- der Glycolipide und der Sphingolipide wie der Ceramide, Cerebroside und Glycosylceramide pflanzlichen oder tierischen Ursprungs.

## Claims

1. A process for the preparation of fractions of fat of vegetable origin enriched with unsaponifiable materials, characterized in that it consists in treating said fat of vegetable origin with a polar solvent of the ketone type in order to recover on the one hand a fraction (I) insoluble in said hot ketone solvent, which constitutes a first fraction rich in unsaponifiable materials, and a solution of a so-called hot-soluble fraction (S) in the ketone solvent, subjecting said hot-soluble fraction (S) to a crystallization step in a so-called crystallization solvent at a temperature below 0°C and filtering the resulting product in order to recover a filtrate (F), which, after evaporation of the crystallization solvent, constitutes a second fraction (I') rich in unsaponifiable materials.

2. The process according to claim 1 characterized in that the treatment with the ketone solvent is carried out at the boiling point of said solvent.

3. The process according to one of claims 1 or 2 characterized in that the ketone solvent is a ketone containing at most ten carbon atoms.

4. The process according to one of claims 1 to 3 characterized in that the ketone solvent is acetone.

5. The process according to one of claims 1 to 4 characterized in that the crystallization step is carried out in any solvent for the fraction soluble in the hot ketone solvent.

6. The process according to claim 5 characterized in that the crystallization step is carried out directly on the solution in the ketone solvent recovered after the step for the treatment of said vegetable fat with said hot ketone solvent.

7. The process according to claim 6 characterized in that the ketone solvent is acetone.

8. The process according to one of claims 1 to 7 characterized in that the crystallization is carried out at a temperature below -15°C, preferably of between -15 and -30°C.

9. The process according to one of claims 1 to 8 characterized in that the starting fat is a fat originating from seeds or fruits of oleaginous plants, obtained by conventional pressure of pressure/extraction techniques, it being possible for said fat to be either crude or refined beforehand.

10. The process according to one of claims 1 to 9 characterized in that the fat is shea butter.

11. The process according to claim 10 characterized in that the shea butter is crude.

12. The process according to claim 10 characterized in that the shea butter has undergone a refining operation for retaining the maximum amount of unsaponifiable material and conserving its constituents.

13. The process according to one of claims 1 to 12 characterized in that it also comprises a step for mixing at least part, preferably all, of each of the two fractions (I and I') rich in unsaponifiable materials.

14. Use of the mixtures of fractions enriched with unsaponifiable materials, obtained by treating a shea butter by the process of claim 13, for the preparation of a cosmetic or pharmaceutical composition, especially dermatological composition, with anti-free radical and/or antielastase activity.

15. The use according to claim 14 characterized in that said composition affords good protection against the conventional aggressions to which the skin is subjected, namely chemical, thermal and chemical aggressions and aggressions resulting from exposure to visible and ultraviolet light radiation.

16. A cosmetic composition containing, as the active ingredient, a cosmetically effective amount of a mixture of fractions enriched with unsaponifiable compounds which is obtained by treating a shea butter by the process described in claim 13.

17. A pharmaceutical composition, especially dermatological composition, containing, as the active ingredient, a pharmaceutically effective amount of a mixture of fractions enriched with unsaponifiable compounds which is obtained by treating a shea butter by the process described in claim 13.

18. The composition according to one of claims 16 or 17 characterized in that said mixture of fractions enriched with unsaponifiable compounds contains 18 to 50% by weight of unsaponifiable compounds, preferably 20 to 50%.

19. The composition according to one of claims 16 to 18 characterized in that said mixtures are incorporated at concentrations of between 0.5 and 99% by weight, preferably of between 2 and 60%, into a cosmetically or pharmaceutically acceptable excipient, vehicle or carrier.

20. The composition according to one of claims 16 to 19 characterized in that it also contains at least one product selected form the group consiting of :
- tocopherols or tocotrienols, especially vitamin E,
- polyunsaturated fatty acids of the n-3 and n-6 series, or oils or plant extracts containing them,
- glycolipids and sphingolipids such as ceramides, cerebrosides and glycosylceramides of vegetable or animal origin.
